# EUROPEAN PATENT APPLICATION

(11) **EP 1 864 650 A1**
(43) Date of publication of application: **12.12.2007**
(21) Application number: 06729979.2
(22) Date of filing: 24.03.2006
(51) Int. Cl.: A61K 9/00

(54) **DOUBLE NEEDLE FOR MEDICAL TREATMENT, BONE CENTESIS NEEDLE, AND BONE MARROW SAMPLING DEVICE**

(30) Priority: 29.03.2005 JP 2005096570
(71) Applicant: NIPPON CABLE SYSTEM INC., Takarazuka-shi Hyogo 665-0845 (JP); Noishiki, Yasuharu, Yokohama-shi, Kanagawa 236-0005 (JP)
(72) Inventor: FUJITA, Yasuhiro c/o HI-LEX Corporation, chome, Takarazuka-shi, Hyogo, 6650845 (JP); AMETANI, Akihiro c/o HI-LEX Corporation, chome, Takarazuka-shi, Hyogo, 6650845 (JP); SEKI, Yasu c/o HI-LEX Corporation, chome, Takarazuka-shi, Hyogo, 6650845 (JP)
(74) Representative: Bailey, David Martin
(86) International application number: PCT/JP2006/306031
(87) International publication number: WO 2006/104060

(57) **Abstract**

To provide a double needle enabling easy centesis in bones and cortex and easy alignment of an inner needle with an outer needle in an angular direction and capable of transmitting torque when the inner needle and the outer needle are rotated simultaneously. This double needle (10) for medical treatment comprises the cylindrical outer needle (11) having an edge part (26) at its tip and the solid inner needle (12) detachably stored in the outer needle. When the outer needle (11) is rotated about its major axis, the rotating diameter of the edge part (26) is larger than the rotating diameter of a body part. Also, the double needle comprises a corotation mechanism (20) for the outer needle (11) and the inner needle (12).

## Description

### Field of the Invention

This invention relates to a double needle, a bone puncture needle, and a bone marrow harvesting device for medical treatments such as bone puncture etc.

### Background Arts

Patent Document 1: Japanese Published Patent Application No. 2003-24339
Patent Document 2: Japanese Utility Model Patent No. H6-21448
Patent Document 3: Japanese Published Patent Application No.2003-116862
Patent Document 4: Japanese Published Patent Application No.2002-28809
Patent Document 5: Japanese Published Patent Application No.2004-154296
Patent Document 6: Japanese Patent No.3311920
Patent Document 7: Japanese Published Patent Application No.2004-290542

For harvesting a bone marrow, a needle is punctured to a hard cortical bone and a catheter etc. is inserted. In this case, a double needle (double puncture needle) which is a combination of a hollow outer needle and an inner needle inserted detachably therein is used. More specifically, the double needle is penetrated from the cortical bone to a bone marrow cavity, then the inner needle is extracted, and a guide wire for guiding the catheter and an exploring tube etc. for inspection is let through. And, there is sometimes a case that the outer needle only is used as the puncture needle without the use of the inner needle.

Patent Document 1 discloses a set of biomedical cement injector comprising a biopsy needle composed of an inner needle and an outer needle for manual puncturing work, an injection tube to inject a biomedical cement, and a stiletto to insert the injection tube after extracting the biopsy needle. The tip of the inner needle and the tip of the outer needle presents a pyramid shape in the state that the inner needle is attached to the outer needle. An inner needle base and an outer needle base are composed so that they form a convexoconcave portion in the contacting face preventing from being out of alignment and to fit mutually.

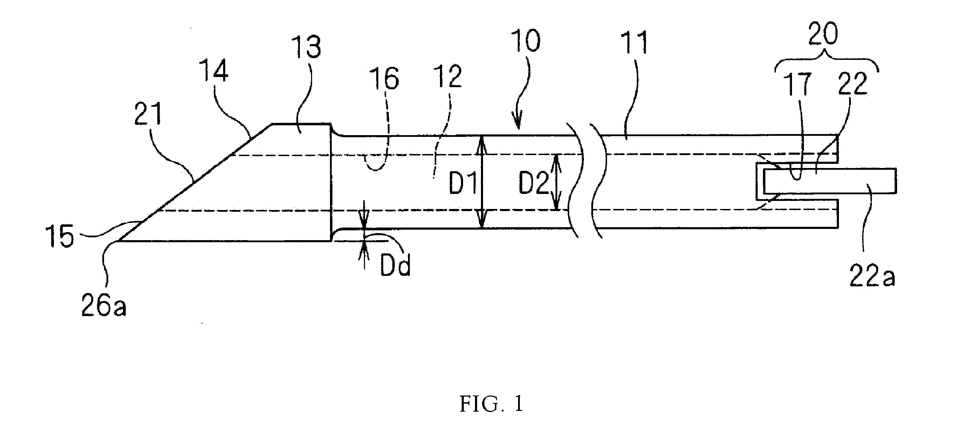

In Patent Document 2, a cell harvesting needle is disclosed, in which an indication (mark) to bring the blade edge faces of the outer needle (outside needle) and the inner needle (inside needle) in line is provided in pairs in the needle base of the inner needle and the needle base of the outer needle. Further, in Figure 1 of Patent Document 2, a composition in which the tip diameter of the inner needle is made thicker than that of the other portion, and a composition in which the blade edge face of the outer needle and the inner needle is made to be an inclined face forming one face are shown.

In Patent Document 3, an electric drill used for harvesting a bone marrow is disclosed, in which an inner needle is housed detachably and rotatably in a cylindrical outer cover. In this document, a preventing means of corotation (a function opposite to a detent) to prevent the corotation of the outer cover and the inner needle is disclosed. Further, it also discloses a drill blade formed in the tip of the inner needle and a spiral groove discharging chips formed in the periphery. In Patent Document 4, although it is not for puncturing a bone of living body, a technology to facilitate the discharge of chips by enlarging the diameter of the tip is disclosed, in a technology of a motor driven compact drill used for metal processing etc.

In Patent Document 5, a soft drill unit composed of a soft tube having flexibility and a soft drill having flexibility inserted therein is disclosed. The soft drill is composed of a flexible wire and an end mill provided in the front end of the wire. The rear end of the wire receives a rotative drive of a motor.

In Patent Document 6 and Patent Document 7, a syringe needle devised in the shape of a needle tip is disclosed.

### Disclosure of invention

The outer needle base and the inner needle base of Patent Document 1 serve also as a handle to corotate the outer needle and the inner needle. The convexity and the concavity are made to be fitted when they are superposed. Hence, the positioning is troublesome. Further, although it is suited for manual labor, it is difficult to connect to the power source when rotated by an electric power. The cell puncture needle of Patent Document 2 is provided with a mark to bring in line the blade edge face of the outer needle and the inner needle, but it needs to carry out the positioning while seeing the mark, and furthermore there is a possibility to become out of alignment gradually while in use.

In the cell puncture needle of Patent Document 2, the rear of the inner needle is thinned, but the outer needle itself is continued to be the same diameter. Hence, the chips cut by the outer needle are hard to be discharged. In Patent Document 3 and Patent Document 4, a drill and an outer cover to guide the drill are equipped. In Patent Document 4, the front edge of the drill is thinned, which makes the chips to be discharged easy. However, any of the cases is not for puncturing cortical bone etc. and used by integrating the inner needle and the outer needle. Patent Document 5 is regarded as a supporting device for bone marrow harvesting which can carry out the puncture smoothly, and it shows future direction of the bone marrow harvesting operations. The future double needles are desired to address such devices.

The syringe needle of Patent Document 6 and Patent Document 7 are needles to stick a soft skin, they cannot be employed as puncture needles to form a deep hole while cutting a bone, but their artifices of the shape of the needle tip give suggestions for improving the tip of the puncture needles.

This invention is directed to provide a double needle and a bone puncture needle which are easy to discharge chips and have a small frictional resistance during the puncturing. Further, this invention is directed to provide a double needle and a bone puncture needle which can address the devices of the type which puncture by motor drive.

### Means for solving the problems

The double needle for medical treatment of this invention (claim 1) comprises a outer needle having a pipe shape and having a body portion and a blade edge portion at a front edge of the body portion, and a solid inner needle detachably housed in the outer needle, wherein the rotating diameter of the blade edge portion is larger than the rotating diameter of the body portion when the outer needle is rotated about its long axis.

In such double needle for medical treatment, the tip of the outer needle is preferable to be tilted toward the rotation axis or to the vicinity of the rotation axis in the long axis of the outer needle (claim 2). Further, it is more preferable to comprises a corotation mechanism to corotate the outer needle and the inner needle when the outer needle is fitted with the inner needle and when the outer needle is rotated about the long axis (claim 3).

In either case, it is preferable that the outer needle and the inner needle are relatively movable along the long axis, and comprises an engaging means to engage the outer needle and the inner needle in the rotative direction (claim 4).

Further, a mark showing the direction of the blade edge face is preferable to be provided in the outer needle (claim 5).

The bone puncture needle for medical treatment of this invention (claim 6) has a body portion having a pipe shape, and a blade edge portion equipped in a front edge of the body portion, wherein the rotation diameter of the blade edge portion is larger than the rotation diameter of the body portion when the outer needle is rotated about its long axis. In such bone puncturing needle for medical treatment also, the blade edge portion of the outer needle is preferable to be brought near to or tilted toward the rotation axis or the vicinity of the rotation axis in the long axis of the bone puncture needle (claim 7). Further, a mark showing the direction of the blade edge face is preferable to be provided in the body portion (claim 8).

The second embodiment of the bone puncture needle of this invention has a body portion having a pipe shape and a blade edge portion trimmed with an inclined plane at a vicinity of a front edge of the body portion, further the sharpening portion trimmed with a plane other than the inclined plane is provided at the front edge of the blade edge portion (claim 9). In such bone puncture needle, the sharpening portion provided at the front edge of the blade edge portion is trimmed with a plane perpendicular to the longitudinal axis (claim 10). Here, the expression "trimmed" includes not only the case carrying out the cutting actually but also carrying out the shape processing by other processes such as grinding by grinder, cutting by press work, forging, or cutting by laser.

And, the front edge of the blade edge portion may be provided with a sharpening portion trimmed with a plane inversely inclined to the above inclined plane (claim 11). Further, the vicinity of the front edge presents a crashed cylindrical shape so as to have a cross section of rough ellipse and the above inclined plane may be somewhat inclined to the plane including the longitudinal axis of the cylinder and the long axis of the cross section (claim 12).

In any of the above puncturing needle, an aspiration port mounting portion to mount an aspiration port may be preferably provided in the vicinity of the rear (claim 13). And, a drill-engaging portion having cross section of a non-circular shape for attaching a mechanical drill at a periphery of the body portion may be provided (claim 14).

The bone marrow harvesting device of this invention comprises a bone puncture needle being punctured into a bone marrow cavity, and an adapter detachable to the bone puncture needle for attaching an aspiration syringe to the bone puncture needle (claim 15). An adapter of this invention to communicate the bone puncture needle with the aspiration syringe comprises a mounting portion being detachably mounted to the bone puncture needle puncturing the bone marrow cavity, and a mounting portion being mounted to the aspiration syringe (claim 16),

In the above described bone marrow harvesting device, it is preferable to further comprises a guide wire puncturing into a bone marrow cavity prior to the puncturing of the bone puncture needle. The guide wire punctures a hole thinner than the bone puncture needle, and guides the bone puncture needle to the bone marrow cavity when puncturing the bone puncture needle (claim 17).

The second embodiment of the bone marrow harvesting device of this invention comprises a double needle punctures into a bone marrow cavity having a outer needle of a pipe shape and an inner needle of a stick shape housed in the outer needle, and an adapter detachable to the outer needle for attaching an aspiration syringe to the outer needle (claim 18).

The third embodiment of the bone marrow harvesting device of this invention comprises a bone puncture needle to be punctured into a bone marrow cavity, a catheter to aspirate the bone marrow, and a guide wire to guide the catheter to the bone marrow cavity along the formed through hole to the bone marrow cavity (claim 19). In the above bone puncturing needle of the bone marrow harvesting device, the bone puncture needle may be a double needle composed of an outer needle having pipe shape and an inner needle having stick shape inserted into the outer needle (claim 20).

The puncture needle is preferable to be attachable to a mechanical drill (claim 21). But, it may comprises a handle provided attachably on the bone puncture needle for rotating the bone puncture needle manually (claim 22).

In the bone marrow harvesting device equipped with a guide wire, the tip of the guide wire is preferable to be bent or curved so as to allow the guide wire to turn around in a bone marrow cavity (claim 23). And, the tip of the guide wire is preferable to be formed to be rough spherical (claim 24).

In the bone marrow harvesting device provided with a catheter, the catheter is preferable to be provided with a first lumen to feed liquid in a bone marrow cavity and a second lumen to aspirate a bone marrow from the bone marrow cavity (claim 25). And, it may be a catheter comprised of one lumen for only aspirating the bone marrow from the bone marrow cavity.

The method for harvesting the bone marrow of this invention comprises, puncturing a Kirschner wire into a bone marrow cavity, inserting a catheter which also serves as a needle into the bone marrow cavity with the guide of the Kirschner wire, extracting the Kirschner wire leaving the catheter, and aspirating the bone marrow through the catheter (claim 26). And, the Kirschner wire and the catheter serving also as a needle may be used by coupling together. Further, when puncturing into the depth of a bone marrow cavity, it is preferable to make the Kirschner wire precede and then puncture the catheter serving also as a needle.

The forth embodiment of the bone marrow harvesting device of this invention comprises a Kirschner wire, a catheter serving also as a needle guided by the Kirschner wire, a coupling means to coupling the Kirschner wire and the catheter so as not to rotate mutually, and an aspirator coupled to the catheter serving also as a needle (claim 27).

### Effect of the invention

In the double needle of this invention (claim 1), the rotating diameter of the blade edge portion of the outer needle is larger than the rotating diameter of a body portion. Hence, there is a clearance between the inner surface of the hole formed in a bone and the body portion when the double needle is punctured while being rotated. Therefore, the friction generated in the body portion will be small and the puncturing work is easy. Further, the space between the inner surface of the hole and the body portion serves as the channel for pushing out the punctured scrap to the outside which also ease the puncturing work.

In the double needle for medical treatment in which the tip of the outer needle is brought near to or tilted toward the rotation axis or to the vicinity of the rotation axis in the long axis of the outer needle, the internal cavity of the outer needle will be occupied by the front edge of the inner needle when the inner needle is inserted into the outer needle. So, the punctured scrap or chips cannot get into the clearance between the outer needle and the inner needle. Moreover, since it rotates about the needle tip (the tip of the outer needle) which is brought near to the center axis, the deviation of the needle tip is small and the safe puncture to the aimed position is possible.

Further, in the double needle further comprising a corotation mechanism to corotate the outer needle and the inner needle when the outer needle is attached with the inner needle and when the outer needle is rotated about the long axis (claim 3), the relative angle position of both the outer needle and the inner needle is determined by the corotation mechanism when the inner needle to the outer needle are attached. Thereby, the positioning is easy. Further, the inner needle and the outer needle can be rotated together just by rotating the outer needle.

When the outer needle and the inner needle are relatively movable along the long axis and an engaging means is provided to engage the outer needle and the inner needle in the rotative direction (claim 4), it is possible to position the tip of the outer needle and the inner needle each other in the angle direction, only by inserting the inner needle straight into the outer needle. Further, the engaging means can be used as the corotation mechanism.

When a mark showing the direction of the blade edge face is provided in the outer needle (claim 5), the outer needle is easy to be inserted into the inner needle in the correct direction during the attachment of the inner needle to the outer needle. Further, the direction of the needle tip (blade edge face) can be confirmed and can be directed or pointed to the safe direction after puncturing the needle.

In the bone puncture needle for medical treatment of this invention (claim 6), the rotation diameter of the blade edge portion is larger than the rotation diameter of the body portion. Hence, when the needle is punctured while being rotated, there is a clearance between the inner surface of the hole formed in a bone and the body portion. Therefore, the friction generated in the body portion is small, thereby the puncturing work and the extracting work of the needle is easy. Moreover, the clearance between the inner surface of the hole and the body portion serves as a passage or a channel to discharge the chips outside making the puncturing work easy.

In the bone puncture needle, in which an inclined plane is formed in the blade edge portion and the tip of the inclined plane is brought near to the rotation axis or to the vicinity of the rotation axis in the long axis of the needle (claim 7), the deviation of the needle tip is small enabling safe puncture to the aimed position, since it rotates about the needle tip.

In the bone puncture needle in which a mark showing the direction of the blade edge face is provided in the body portion (claim 8), it is possible to check the direction of needle tip (blade edge face) and it is possible to point the needle tip to a safe direction.

In the second embodiment of the bone puncture needle of this invention (claim 9), in addition to the fundamental shape of the blade edge portion scraped at a slant with an inclined plane, a sharpening portion trimmed with a plane other than the inclined plain is provided at the front edge of the blade portion. Therefore, it is possible to cut cortical bones etc. efficiently in accordance with the shape of the blade edge portion, and it is possible to prevent the puncturing of the opposite side of the cortical bones of the bone marrow cavity and to prevent the breaking or dropping out of the thin-walled portion.

When the front edge of the blade edge portion is provided with a sharpening portion scraped perpendicular to the longitudinal axis (claim 10), it can prevent the puncturing or penetrating of the opposite side of the cortical bone to the bone marrow cavity, when puncturing a bone while rotating the bone puncture needle. Moreover, since its front edge is not sharpened, the possibility of this portion to drop out is reduced.

When the front edge of the blade edge portion is provided with a sharpening portion scraped with a plane inversely inclined to the above inclined plane (claim 11), the hollowing out of the opposite side of the cortical bone can be prevented without degrading the puncturing performance.

In the third embodiment of the puncture needle of this invention (claim 12), since the vicinity of the front edge presents a crashed cylindrical shape so as to have a rough ellipse cross section and the above inclined plane is somewhat inclined to a plane including the longitudinal axis of the cylinder and the long axis of the cross section, the edge of the one side can cut tissues while cutting a cortical bone by rotation. Thereby, the cutting efficiency is high.

In any of the above bone puncture needle having an aspiration port mounting portion to mount an aspiration port in the vicinity of the rear (claim 13), the bone puncture needle itself can serve as an aspiration catheter. Hence, the required parts can be reduced allowing easy bone marrow harvesting. When a drill-engaging portion of a non-circular cross section so as to be attached to a mechanical drill is provided in the periphery (claim 14), the bone puncture needle can be efficiently rotated when attached to an electric mechanical drill or a hand mechanical drill.

The bone marrow harvesting device of this invention (claim 15) comprises a bone puncture needle being punctured into a bone marrow cavity, and an adapter detachable to the bone puncture needle so as to attach an aspiration syringe to the puncture needle. Therefore, after puncturing the puncture needle to a bone marrow, an aspiration syringe is attached to the bone puncture needle through the adapter, and the bone marrow can be aspirated as it is without replacing the catheter. And, since the adapter is detachable to the puncture needle, the puncture needle can be used for other applications in place of the aspiration syringe such as for the injection of diluted solution of physiologic saline etc.

The adapter of this invention (claim 16) can connect the puncture needle and the aspiration syringe by coupling the puncture needle punctured to a bone marrow cavity to the mounting portion of the puncture needle and by attaching the syringe to the mounting portion of the syringe. Hence, the bone marrow can be aspirated as it is after the puncturing needle is punctured to the bone marrow cavity, by attaching the syringe to the puncture needle through the adapter without replacing with the catheter. And, since the adapter of this invention is detachable to the puncture needle, the puncture needle can be used for other usage such as injection of diluted solution of physiologic saline etc. in place of the aspiration syringe.

In the above described bone marrow harvesting device, in which further comprises a guide wire puncturing into a bone marrow cavity prior to the bone puncture needle, and which the guide wire punctures a hole thinner than the bone puncture needle and guides the bone puncture needle to the bone marrow cavity when puncturing the bone puncture needle (claim 17), the device is guided to a bone marrow cavity by the guide wire at the beginning, and the device can puncture by the puncture needle along the guide wire. Thereby, an accurate puncture path can be formed.

The second embodiment of the bone marrow harvesting device of this invention (claim 18) is provided with a double needle punctured into a bone marrow cavity having a outer needle with a pipe shape and an inner needle with a stick shape housed in the outer needle, and an adapter detachable to the outer needle for attaching an aspiration syringe to the outer needle. In this case, the inner needle is punctured precedently, and then the outer needle can be punctured along the inner needle. After the penetration to the bone marrow cavity, the inner needle is removed, and the aspiration syringe is attached to the outer needle through the adapter, so the bone marrow can be aspirated by making the outer needle act as a catheter.

In the third embodiment of the bone marrow harvesting device of this invention (claim 19), the bone marrow can be aspirated through the catheter after forming a through hole to the bone marrow cavity, letting a guide wire into the through hole, letting a catheter into the bone marrow cavity with the guide of the guide wire.

When the bone puncture needle of the bone marrow harvesting device is made to be a double needle composed of an outer needle having a pipe shape and an inner needle having stick shape inserted therein (claim 20), a thin through hole can be formed previously by the inner needle without the use of the guide wire or the Kirschner wire. The forming of the through hole may be carried out with a state of enhanced rigidity by joining the inner needle and the outer needle

Further, when the puncture needle is made to be attachable to a mechanical drill (claim 21), puncturing may be carried out efficiently by an electric mechanical drill or a hand mechanical drill. Moreover, when a handle to rotate the puncture needle is detachably provided in the bone puncture needle (claim 22), the bone puncture needle can be rotated by the handle, thereby the efficient puncturing can be carried out with a simple composition.

The bone marrow harvesting device equipped with a guide wire in which the tip of the guide wire is bent or curved for allowing the guide wire to turn around in a bone marrow cavity (claim 23) is capable of changing the direction of the front edge of the guide wire freely by rotating the guide wire after the front edge of the guide wire reaches a bone marrow cavity. Thereby, the bone marrow to be aspirated can be gathered together from a wide region. And, when the tip of the guide wire is formed to be rough spherical (claim 24), the damaging of inner wall of the bone marrow cavity by the tip of the guide wire can be prevented.

The bone marrow harvesting device equipped with a catheter in which the catheter is provided with a first lumen to feed liquid in a bone marrow cavity and a second lumen to aspirate a bone marrow from the bone marrow cavity (claim 25), it is possible to make solvent such as physiologic saline etc. flow in from the first lumen while diluting the bone marrow and to aspirate/harvest the diluted bone marrow through the second lumen efficiently. In addition, even when the catheter having only one lumen is used, after the bone marrow is somewhat aspirated, the physiologic saline may be made to flow in to dilute the bone marrow, and further, the diluted bone marrow can be aspirated from the lumen.

The method for harvesting the bone marrow of this invention (claim 26), comprises method of inserting a catheter which also serves as a needle (needle catheter) into the bone marrow cavity with the guide of the Kirschner wire, after a Kirschner wire is punctured into the bone marrow cavity. Since, the Kirschner wire is accustomed to use by surgeons, the pouncture work can be done easy. Further, since the needle catheter is used, the work to substitute the puncture needle with the catheter is not necessary. More specifically, the necessary methods in which the puncture needle is substituted with the catheter for aspiration are, puncturing the bone puncture needle along the Kirschner wire, extracting the bone puncture needle, inserting the catheter, extracting the Kirschner wire, and aspirating the bone marrow after inserting the catheter. Further, depending on the circumstances, extracting of the Kirschner wire and substituting of the Kirschner wire with the guide wire, and inserting of a catheter for aspiration may also be necessary. In the harvesting method of this invention, since the catheter serving also as a needle is used, the above described method can be omitted enabling to make the bone marrow harvesting work more efficient. More, the parts management is lessened due to the reduction of the used parts. When the Kirschner wire and the needle catheter is used by being coupled, the required length of the Kirschner wire when puncturing to the far end of a bone marrow cavity may be shortened enables easy handling. Further, by advancing the needle catheter and the Kirschner wire together, the chips are prevented from entering the internal cavity of the needle catheter.

Since the forth embodiment of the bone marrow harvesting device of this invention (claim 27) comprises a Kirschner wire, a needle catheter guided by the Kirschner wire, a coupling means to couple them together so as not to rotate mutually, and an aspirator connected to the catheter, the above described bone harvesting method of this invention can be carried out.

### Brief Description of Drawings

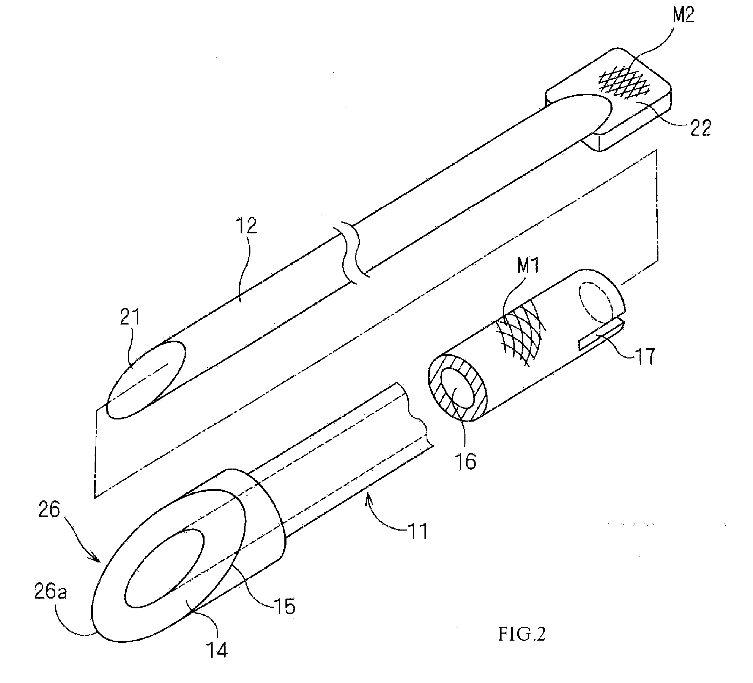

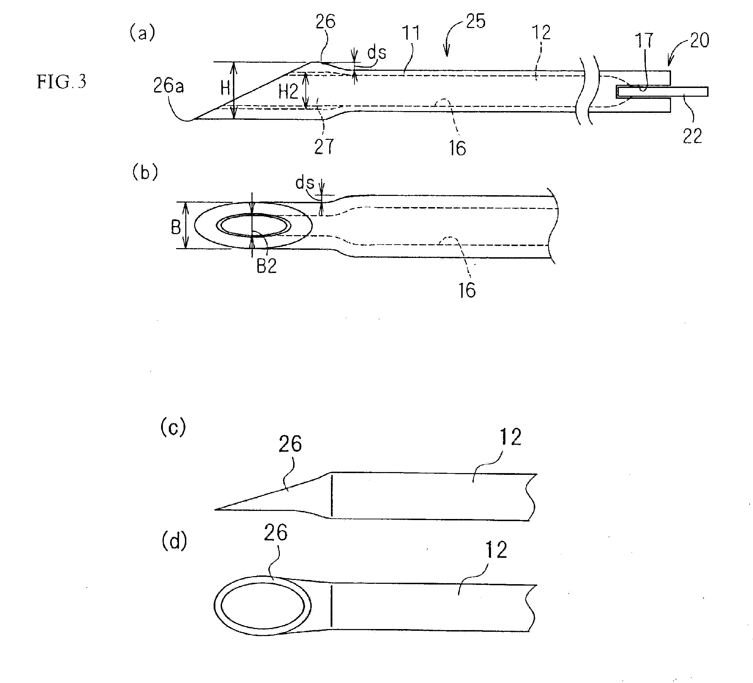

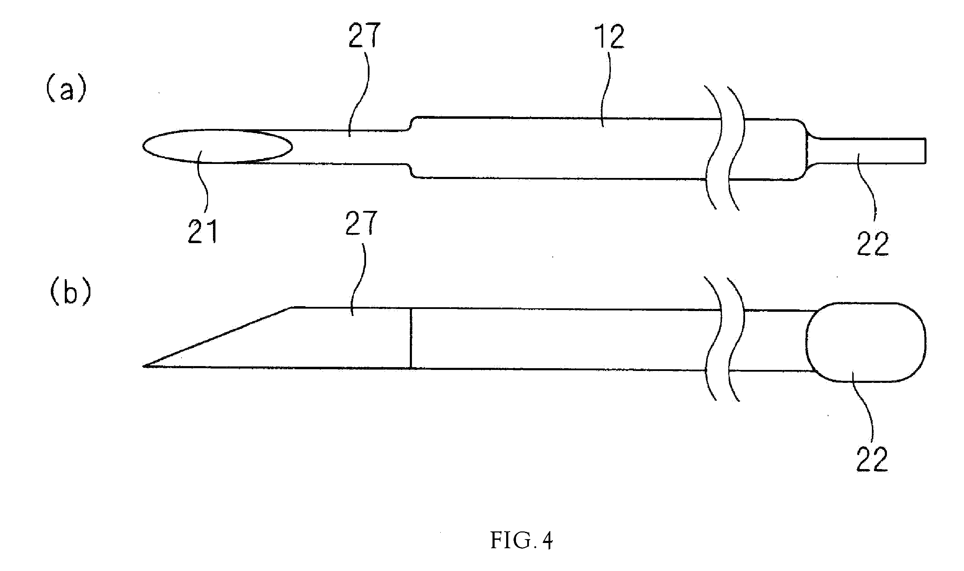

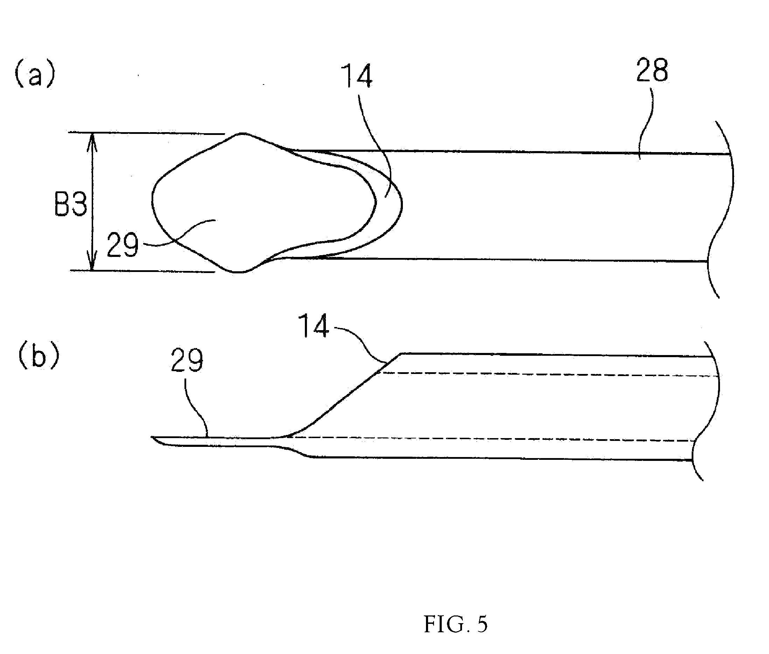

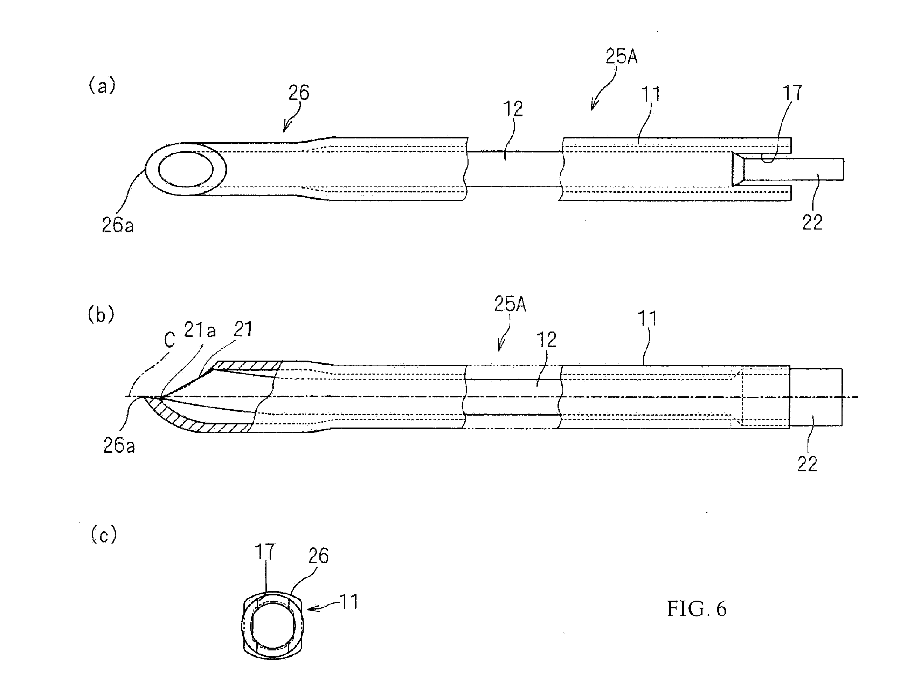

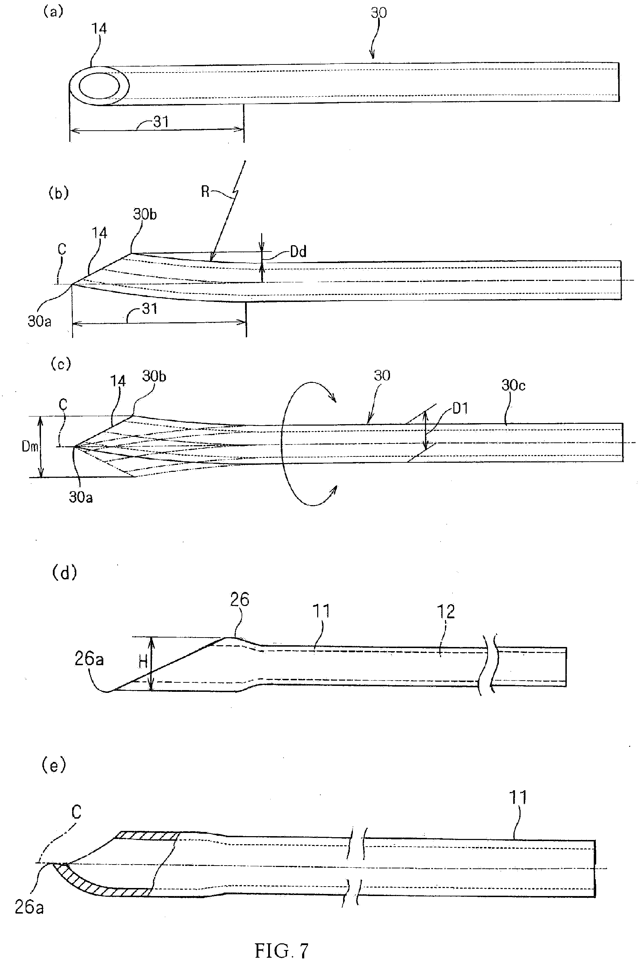

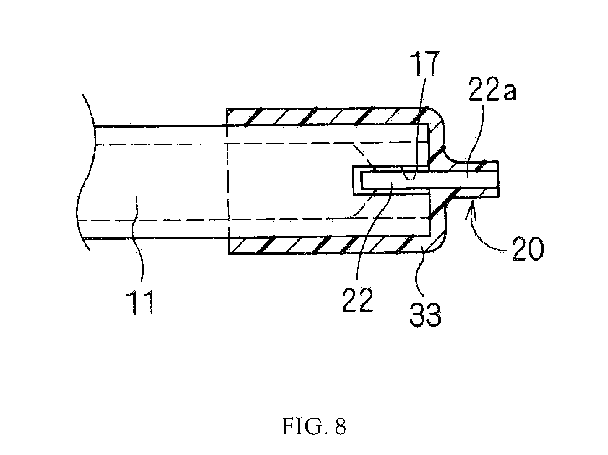

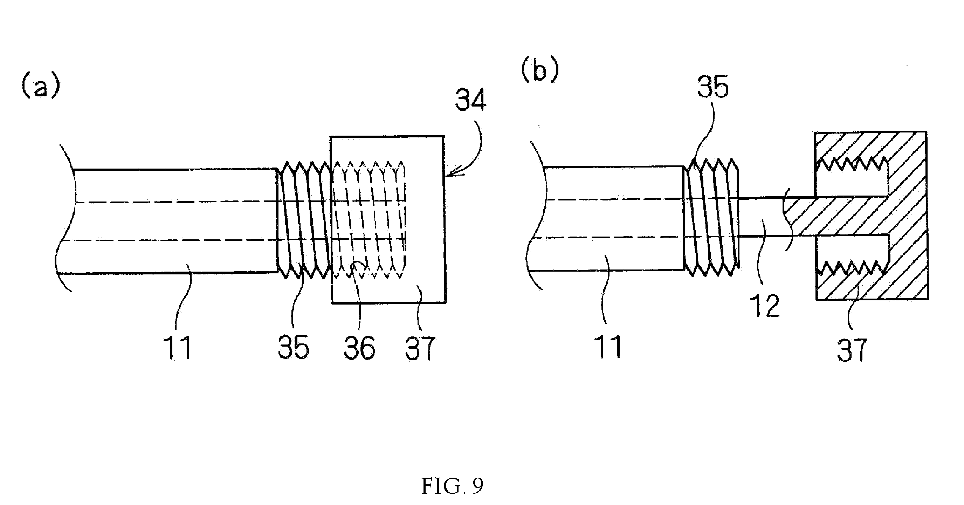

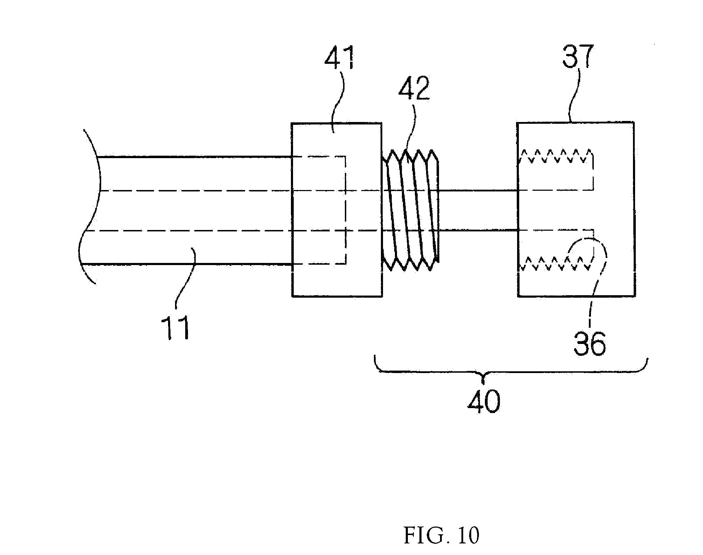

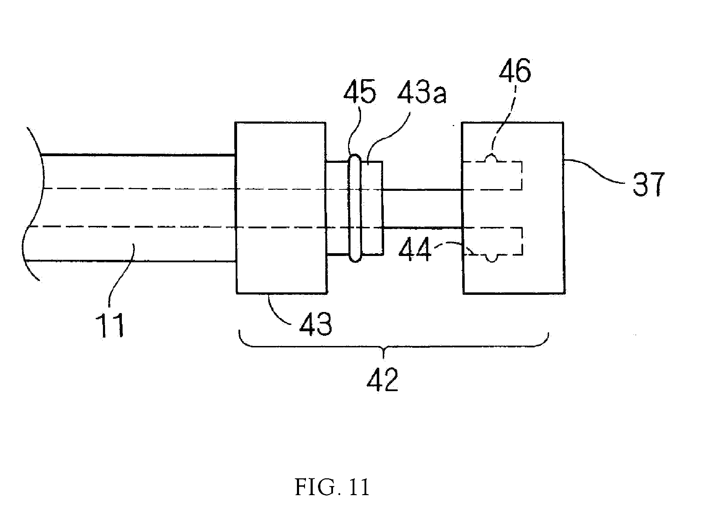

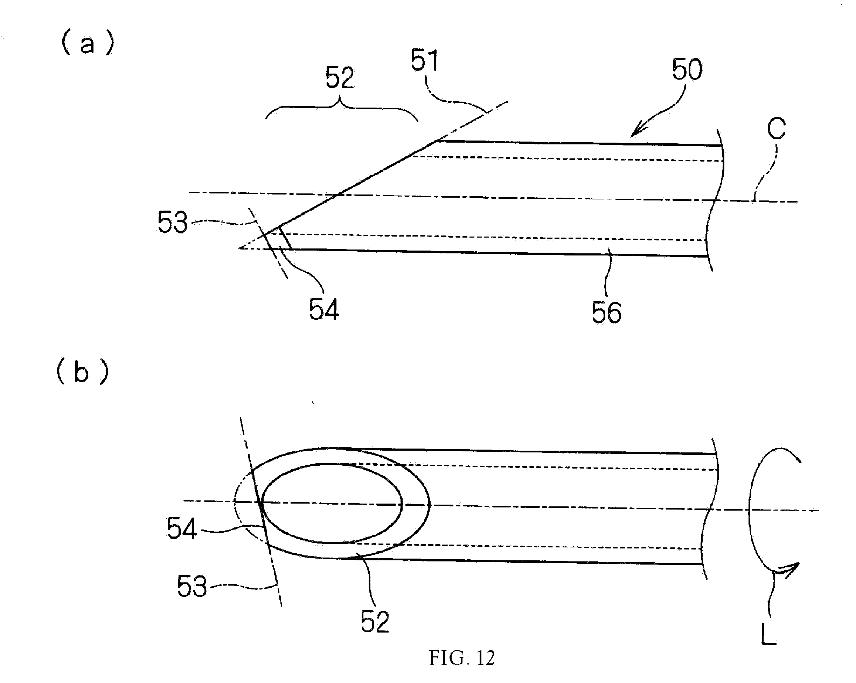

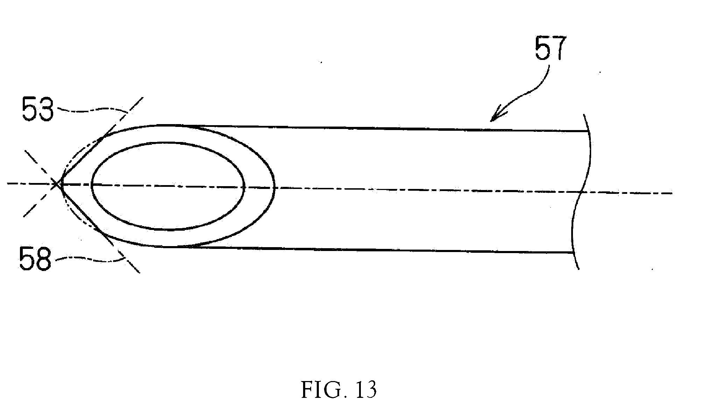

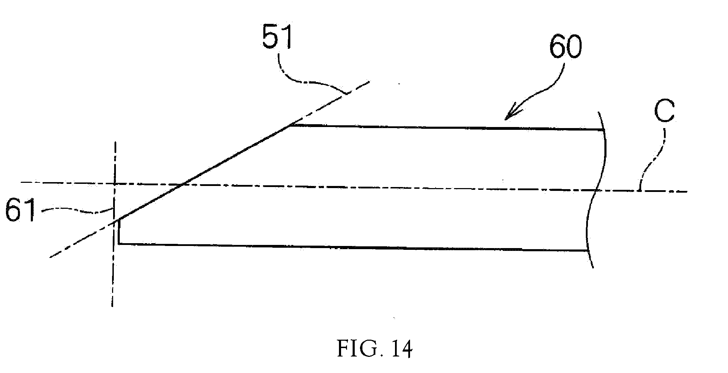

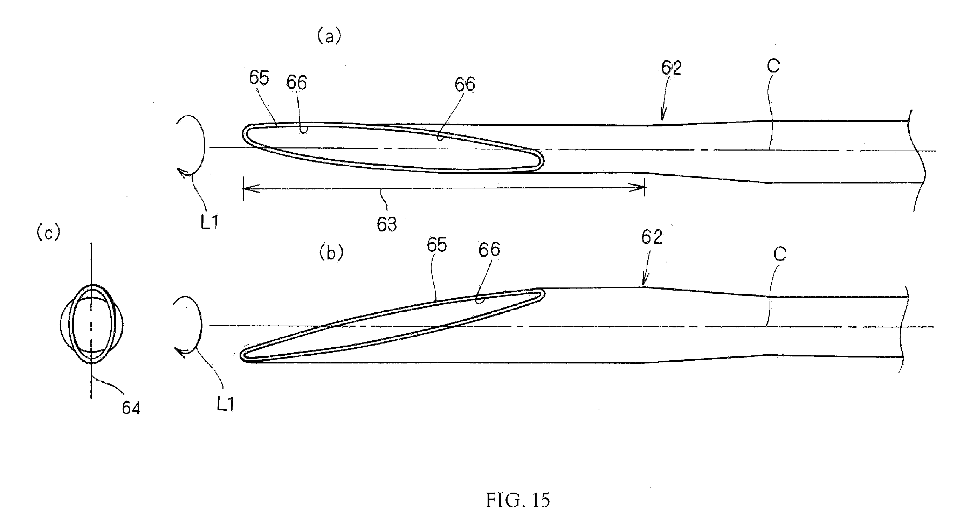

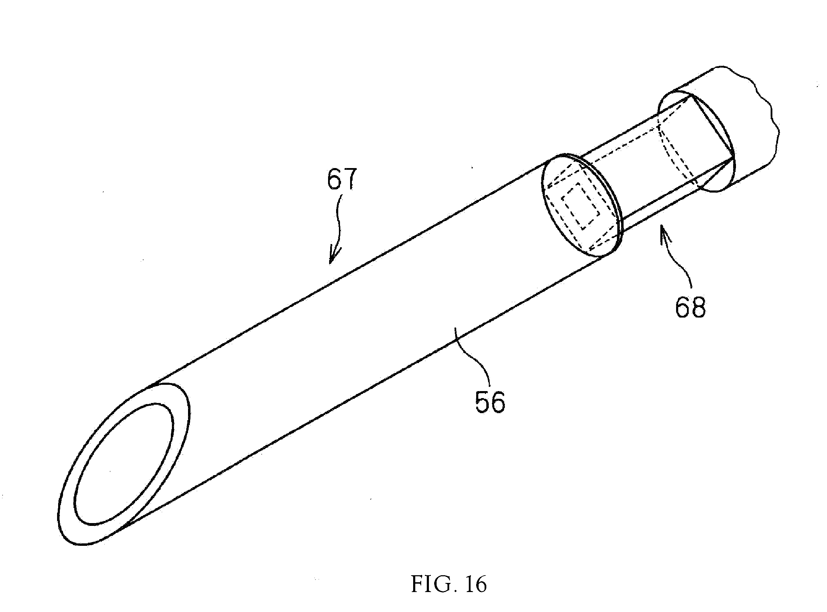

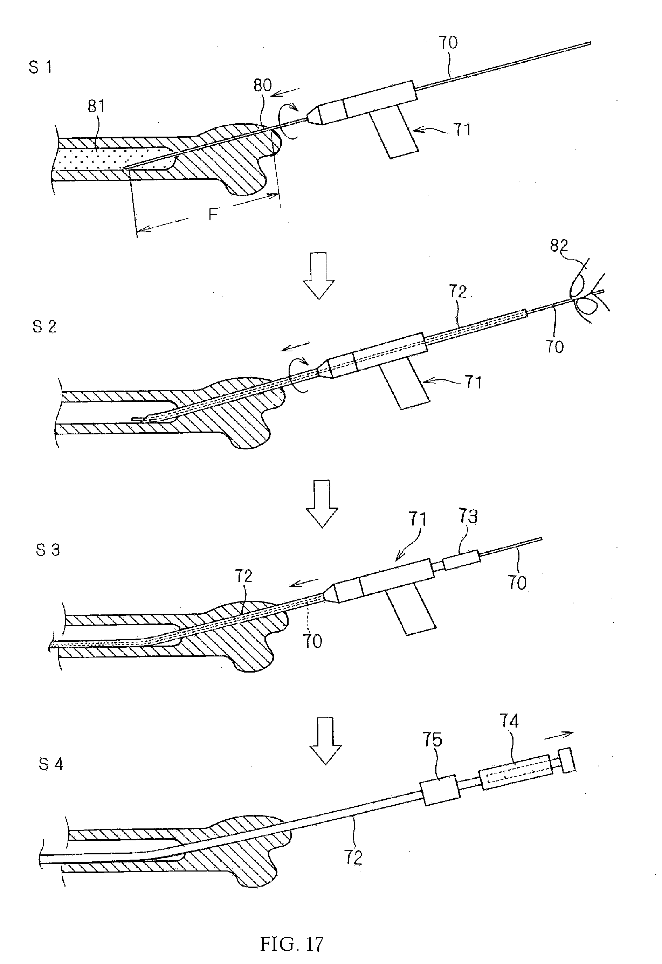

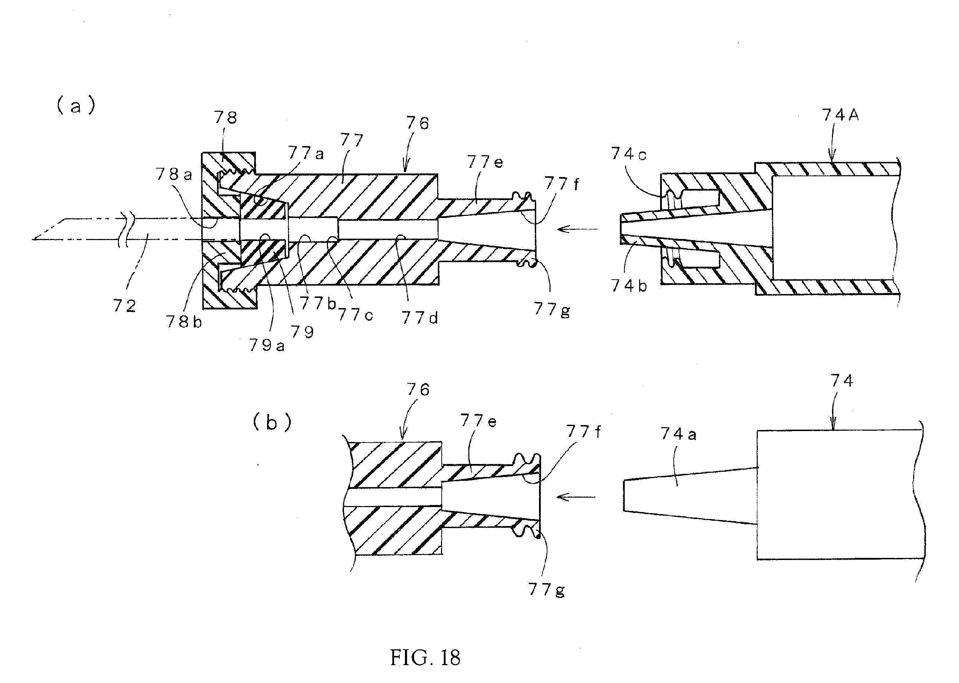

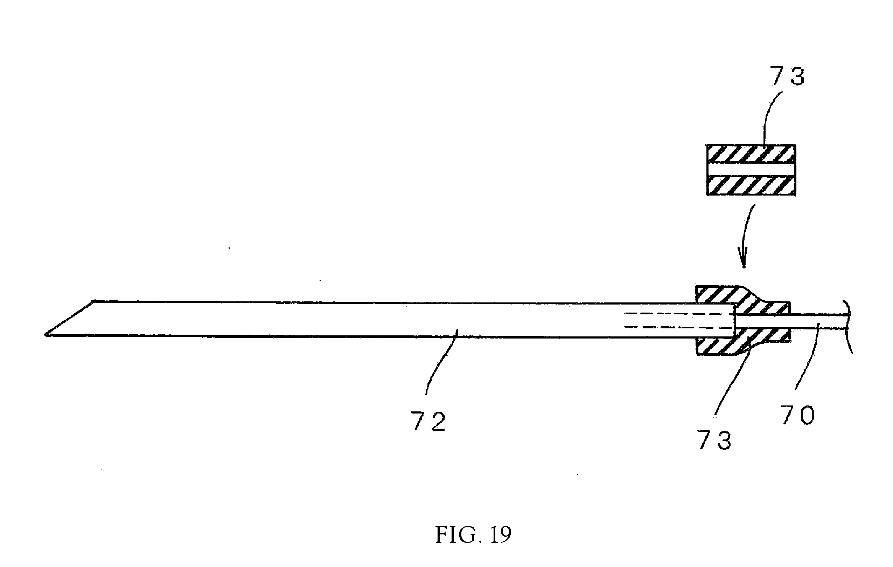

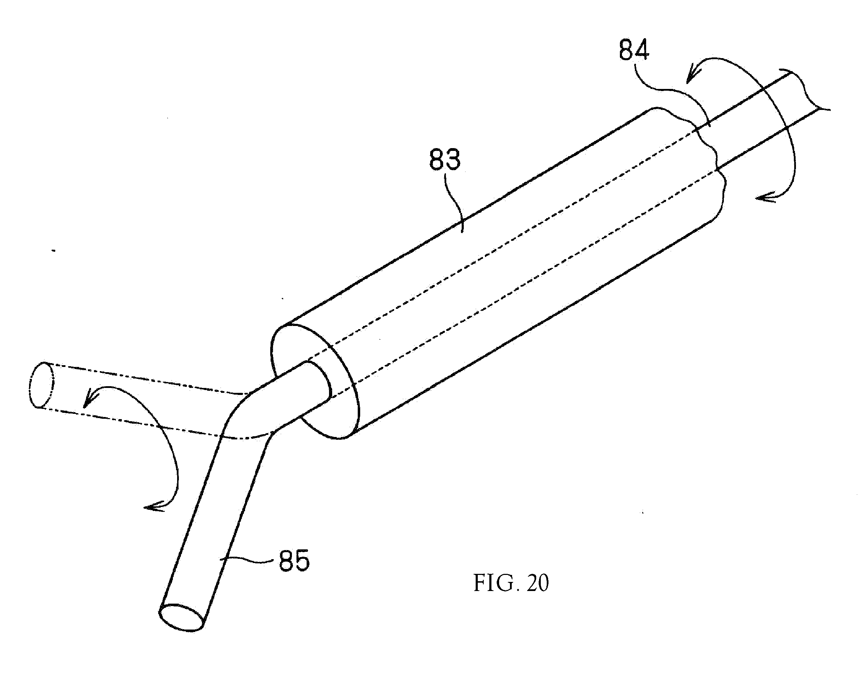

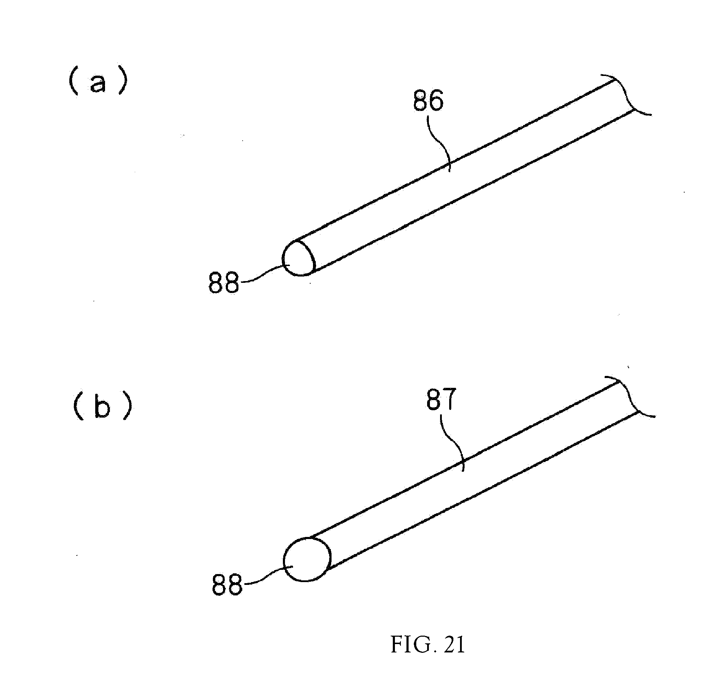

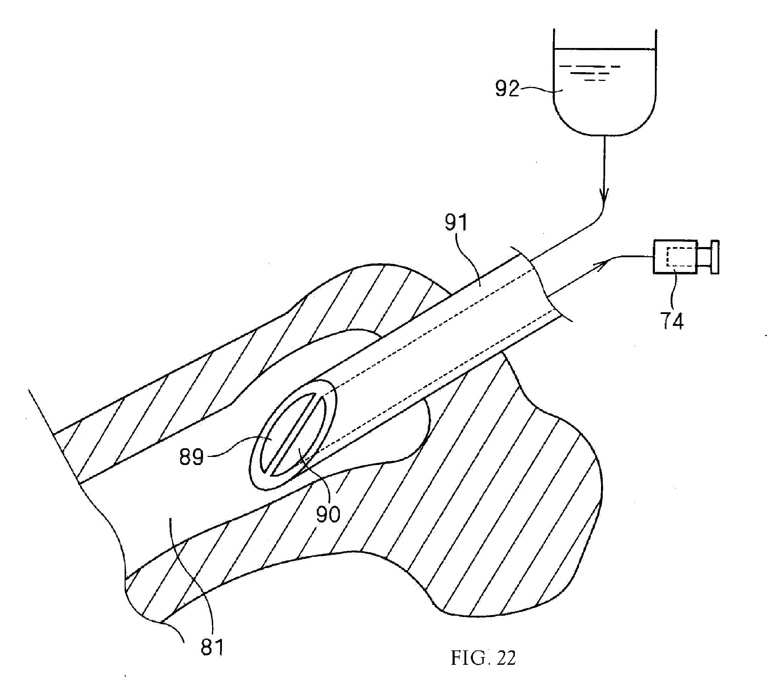

Figure 1 is a side view showing an embodiment of the double needle of this invention; Figure 2 is an exploded perspective view of the double needle; Figure 3a and Figure 3b are a side view and a plane view respectively showing the other embodiment of the double needle of this invention, Figure 3c and Figure 3d are a side view and a plane view respectively showing further the other embodiment of the double needle of this invention; Figure 4a and Figure 4b are a plane view and a side view respectively related to the inner needle of this invention; Figure 5a and Figure 5b are a plane view of the essential part and a side view of the essential portion respectively showing further the other embodiments of the outer needle related to this invention; Figure 6a and Figure 6b are a plane view and a partially notched side view respectively showing further the other embodiment of the double needle of this invention, Figure 6c is a back view showing the rear side of the outer needle of this invention; Figure 7a and Figure 7b are a plane view and a side view respectively showing further the other embodiment related to the outer needle of this invention, Figure 7c is a side view showing the action of the outer needle, Figure 7d and Figure 7e are side views showing the embodiment of the puncture needle of this invention; Figure 8 is a side view showing an embodiment of the temporal fixing method related to this invention; Figure 9a and Figure 9b are side views respectively showing the status being temporally fixed and the status being released in the temporal fixing structure in the other embodiment related to this invention; Figure 10 is a side view showing further the other embodiment of the temporal fixing structure related to this invention; Figure 11 is a side view showing further the other embodiment of the temporal fixing structure related to this invention; Figure 12a and Figure 12b are a side view and a plane view showing further the other embodiment of the puncture needle of this invention; Figure 13 is a plane view showing further the other embodiment of the puncture needle of this invention; Figure 14 is a side view showing further the other embodiment of the puncture needle of this invention; Figure 15a, Figure 15b, and Figure 15c are a plane view, a side view, a front view respectively showing further the other embodiment of the puncture needle of this invention; Figure 16 is a perspective view of the essential portion showing further the other embodiment of the puncture needle of this invention; Figure 17 is a process drawing showing an embodiment of the bone marrow harvesting method of this invention; Figure 18a is a cross sectional view showing an embodiment of an adapter of this invention, and Figure 18b is a cross sectional view of the essential portion showing the other usage of the adapter; Figure 19 is a cross sectional view showing an embodiment of the coupling means related to this invention; Figure 20 is a perspective view showing an embodiment of the guide wire of this invention; Figure 21a and Figure 21b are perspective views showing further the other embodiment of the guide wire of this invention; Figure 22 is a rough side view showing the embodiment of the catheter related to this invention.

### Best mode for carrying out the invention

The double needle 10 shown in Figure 1 comprises an outer needle 11 having cylindrical shape and an inner needle 12 inserted into the inside of the outer needle. The vicinity of the front edge of the outer needle 11 is made to be a large diameter portion 13 whose outer diameter is larger than that of the other portion (body portion). Thereby, the rotation diameter of the large diameter portion 13 is larger than the rotation diameter of the other portion. And, the circle drawn by the rotation diameter of the other portion becomes to be within the circle drawn by the rotation diameter of the large diameter portion. The tip of the outer needle 11 is made to be an inclined plane 14. Thereby, the peripheral edge 15 of the inclined plane 14 acts as a cutting blade. In the rear end of the outer needle 11, an engaging groove 17 extending toward radial direction and reaching an internal cavity 16 is formed. The cutting blade can be of the shape publicly known such as pyramid, saw-tooth other than the inclined plane. Providing the large diameter portion 13 allows to lessen the grinding area and to narrow the friction area with bones.

Such outer needle 11 can be manufactured by press forming a metal pipe having cross section of circle, for example, stainless, nitinol. The large diameter portion 13 of the vicinity of the front edge can be manufactured by deformation processing such as diameter-enlarging processing, but it may be formed by cutting the portion other than the vicinity of the front edge. The inclined plane 14 can be formed by cutting and grinding. In addition, it can be manufactured by shaping a metal plate into cylindrical shape. The outer diameter D1 of the body portion of the outer needle 11 is preferable to be, for example, about 1-6mm, particularly about 2-4mm. The step Dd between the large diameter portion 13 and the other portion (body portion) is preferable to be about 0.1-0.3mm. The angle of the inclined plane 14 is preferable to be 20-60 degrees, particularly about 20-40 degrees. The length of the outer needle is, about 50-500mm though different by the object of use, and when it is used as a puncture needle for bone marrow harvesting, for example, length of about 100-200 mm is used.

The inner needle 12 is a stick shape member having circular cross section, and an inclined plane 21 having the same angle of inclination with the outer needle 11 is formed in its tip as shown in Figure 2. Thereby, when the inner needle 12 is inserted into the outer needle 11, the inclined plane 14, 21 mutually becomes one plane as shown in Figure 1. Further, in the rear end of the inner needle 12, a plate-like flat portion 22 engaging with a groove 17 of the outer needle 11 is formed. The flat portion 22 is preferable to be formed by press forming a stick shape material which is the material of the inner needle 12.

Such inner needle 12 can be manufactured by press forming the metal wire to form the flat portion 22 and by cutting and grinding to form the inclined plane using the material similar to that of the outer needle 11, preferably a metal wire having high rigidity and high flexibility, for example, spring steel wire, stainless wire, metal wire such as nitinol (nickel/titanium system shape memory alloy). The outer diameter D2 of the inner needle 12 is, for example, about 1-3mm, particularly about 1.6-2mm. The internal cavity 16 of the outer needle 11 is approximately same as the diameter of the inner needle 12, and the slidable fit tolerance of about 0.1-0.4mm is provided. Since this tolerance and the shape of the cross section are same, the outer needle 11 and the inner needle 12 are rotatable when the flat portion 22 is not engaged with the groove 17.

As shown in Figure 2, in the rear or the central part of the outer needle 11, a mark M1 is provided in the position corresponding to the inclined plane 14. Hence, the inclined plane 21 of the inner needle 12 is easy to be aligned with the inclined plane 14 of the outer needle 11 by inserting the inner needle 12 into the outer needle 11. Additionally, a mark M2 may be provided on the upper face of the flat portion 22 of the rear end of the inner needle 12 matching with the mark M1 of the outer needle. Thereby, the direction (the direction of front and rear) of the inclined plane 21 of the inner needle is hard to be mistaken.

The width of the flat portion 22 is preferable to be same as the diameter of the outer needle 11 or somewhat narrower than it. Thereby when the inner needle 12 is inserted into the outer needle 11, the flat portion 22 does not protrude from the surface of the outer needle 11. However, the side edge of the flat portion 22 can be protruded. On the other hand, the length of the flat portion 22 is longer than the length of the engaging groove 17. For example, when the length of the engaging groove 17 is made to be 3mm, the length of the flat portion is made to be 7mm. Thereby, when the inner needle 12 is inserted into the outer needle 11 and the front edge of the flat portion 22 engages with the engaging groove 17, the rear 22a of the flat portion 22 protrudes from the rear end of the outer needle 11. The thickness of the flat portion 22 is, for example, about 0.6-1.5mm. The width of the engaging groove 17 of the outer needle 12 is made to be about 0.15-0.25mm larger than that. Thereby, the engaging/release action becomes easy.

In the above double needle 10, since the flat portion 22 of the inner needle 12 engages with the engaging groove 17 when the inner needle 12 is inserted into the outer needle 11, the positioning can be easily done. Further, since they do not rotate relatively, the inner needle 12 and the outer needle 11 do not become out of alignment in the rotative direction. Hence, by attaching the inner needle 12 to the outer needle 11, the inclined planes 14, 21 will be kept mutually in one plane preventing to become out of alignment while at work. In this occasion, the back portion 22a of the flat portion 22 protrudes from the rear end of the outer needle 11. In this state, the handle is attached to the rear end of the double needle 10, and the double needle 10 is operated by hand alternatively moving forward and backward while rotating right and left. Thereby, the tip portion of the double needle 10 can be advanced to a bone marrow cavity puncturing cortical bones. Moreover, the rear end portion of the double needle 10 may be coupled to a rotation shaft etc. using a drill chuck, and the double needle 10 can be operated to puncture with motor drive or turning by hand. The rear portion 22a (depending on the case, side edge) of the flat portion 22 can be used for coupling capable of torque transmission by engaging with a handle or a driving shaft.

When the puncturing is completed, the inner needle 12 is extracted, and a guide wire or a catheter can be passed through the internal cavity 16 of the outer needle 11. In the case where the guide wire is passed through, the outer needle 11 is extracted once, and the catheter is passed through to a bone marrow cavity with the guide of the guide wire, and the catheter can be used for the harvesting of the bone marrow and the inspection of the bone marrow. And, in the case where the catheter can be passed through into the internal cavity 16 of the outer needle 11 directly, the catheter can be used for the harvesting and the inspection of the bone marrow.

In the double needle 25 shown in Figure 3a and Figure 3b, the blade edge portion 26 of the outer needle 11 is not enlarged in its diameter uniformly. Its width is compressed to be narrow, and its height is made to be somewhat high, thereby the blade edge portion 26 is flat being longer than is wide. This processing can be done by applying press working to a metal pipe. The dimension of the each step ds is about 0.1-0.3 mm. The diameter and the wall thickness of the other portion of the outer needle 11 is made to be cylindrical same as the case of Figure 1. But it can be elliptical shape with small longitudinal dimension being long in width. In the case where it is processed by press working, the internal cavity 16 also becomes narrow in its width B2 and higher in its height H2. Thus, by making the height H be higher than that of the body portion, the rotation diameter of the blade edge portion 26 becomes larger than the rotation diameter of the body portion. In addition, as shown in Figure 3c, 3d, the blade edge portion 26 can be pressurized in the vertical direction. In this case also, the rotation diameter of the blade edge portion 26 becomes larger than the rotation diameter of the body portion.

In the double needle 25 shown in Figure 3a and Figure 3b, the width of the vicinity 27 of the tip of the inner needle 12 is made to be narrow being matched with the blade edge portion 26. This processing can be carried out by grinding or by sanding metal wire material, or grinding metal wire after press working. And, it may be a thin wire diameter inner needle being matched with the width B2 of the internal cavity. The height of the vicinity 27 of the tip of the inner needle 12 is left to be the original diameter of the wire material so as to be insertable into the internal cavity 16 of the outer needle 11. Therefore, a clearance will be left between above and below of the inner needle 12 and the outer needle 11, however it is not an obstacle for the puncturing work. Additionally, the clearance can be closed by bending the tip 26a of the blade edge portion 26 so as to bring near to the long axis C like shown in Figure 6b.

In such double needle 25, as shown in Figure 3a and Figure 3b, the inner needle 12 does not rotate in the outer needle, because the vicinity 27 of the tip of the inner needle 12 fits to the blade edge portion 26 of the outer needle whose cross sections are not circle respectively, when the inner needle 12 is inserted into the outer needle 11. Thus, the blade edge portion 26 of the outer needle 11 and the vicinity 27 of the tip of the inner needle 12 respectively formed to be flat, compose a corotation mechanism by themselves. The use or usage of the double needle 25 is same as the case of the double needle 10 of previous Figure 1. In addition, as shown in Figure 3a, the corotation mechanism 20 of the back end side same as the case of Figure 1 can be employed together.

In this case, as shown in Figure 4a and Figure 4b, the direction of flat portion 22 is preferable to be made in the same direction with the vicinity of the tip 27 which is flatten, thereby press working can be carried out at one time. The direction of the engaging groove 17 of the outer needle 11 is matched with the direction of the flat portion 22. In addition, the vicinity of the tip 27 is preferable to be processed by grinding and cutting.

In the outer needle 28 shown in Figure 5a and Figure 5b, the tip is made to be an inclined plane 14, and further the tip portion 29 is compressed to be flat by press working. The width B3 of the tip portion 29 is, for example, 3-3.5 mm, particularly about 3.2mm. The double needle equipped with such outer needle 28 exerts the effect of easy grinding in bone puncturing.

In the double needle 25A shown in Figure 6a and Figure 6b, the outer needle 11 is almost same as the case of Figure 3a and Figure 3b, and has a blade edge portion 26 pressurized in the width direction making its vertical dimension be larger than that of the body portion (see Figure 6c). The tip of the blade edge portion 26 is made not to be a straight inclined plane but to be concave plane in which its inclined plane becomes slow toward the tip, and the tip 26a of the blade edge 26 is brought near to the long axis (center line) C. Further, the vicinity of the blade edge of the inner needle 12 including the inclined plane 21 curves upward along the inner face shape of the outer needle 11 and the tip 21a of the inclined plane is brought near to the long axis C, when the inner needle 12 is inserted as shown in Figure 6b. The inner needle is not purposely processed to be bent. Therefore, the internal cavity of the outer needle 11 closes and denies the pieces of bone marrow in puncturing from entering into the clearance between the inner needle 12 and the outer needle 11. Meantime, the tip 26a of the outer needle 11 and the tip 21a of the inner needle 12 may come to on the long axis C or may come to near thereof. Further, the clearance does not have to be completely closed, an appreciable effect is exerted only by being narrowed.

In the vicinity of the tip 26a of the outer needle 11, the outer face forms a convex plane protruding outward. And, the inner face forms a concave curvature plane such as inside of a spoon in which the original cylindrical plane and the curvature with its inclined plane being slowed toward the tip are combined. Other portions are same as the double needle 25 shown in Figure 3a and Figure 3b. In addition, all the edge of the tip 26a of the blade edge portion 26 of the outer needle 11 presented in the double needle 10, 25, 25A of Figure 2, Figure 3b, Figure 6b, is presented in a circular arc. However it may be sharpen so as to present a sharp angle or an obtuse angle. Since in the double needle 25A of Figure 6a and Figure 6b, the tip (needle tip) 26a of the blade edge portion 26 is brought near to the long axis C and the needle can be rotated about the needle tip, the stable puncture can be carried out contributing to safety.

In the outer needle 11 of Figure 3a, Figure 3b, Figure 6a, Figure 6b, the rotation diameter is made larger than that of the body portion by making the dimension in the width direction of the blade edge portion 26 be small and the dimension in the longitudinal direction be large. But the outer needle may equip with region 31 of the tip side having thickness same as the body portion and simply be curved. In this embodiment, it is curved in the circular arc of the predetermined curvature radius R so as to warp toward the inclined plane 14 side. The tip 30a almost coincides with the long axis C, and the upper end 30b of the inclined plane 14 protrudes from the surface of the body portion with the predetermined step Dd. Hence, as shown in Figure 7c, when the outer needle 30 is rotated around the long axis C, the rotation diameter Dm becomes larger than the diameter D1 of the body portion as much as 2 × Dd. When the diameter of the body portion of the outer needle 30 is 3 mm, the step Dd is usually about 0.5-1mm, and the curvature radius R is about 60-80mm. This outer needle 30 exerts the same effects as the outer needle of Figure 6a, Figure 6b in puncturing, and is easy to manufacture.

The outer needle 30 of Figure 7 can be used not only together with the inner needle as a double needle, but can be used by itself as a bone puncture needle. Moreover, the outer needle 11 of Figure 3a, Figure 3b also can be used as a bone puncture needle. In this case, the engaging groove in the rear end (reference numeral 17 of Figure 3) is not necessary as shown in Figure 7d. The outer needle 11 (bone puncture needle) of Figure 7d can be used together with a Kirschner wire used generally in orthopedic surgery etc. More specifically, since the Kirschner wire has nothing but a blade in the tip of a simple stick, there is a resistance in puncturing and extraction. But the outer needle (bone puncture needle) can assist the puncture of the Kirschner wire utilizing the feature of easy puncture and extraction. In the case where both are employed, the outer needle 11 or the Kirschner wire may be used in precedence. When the outer needle is used in precedence, the chips may be clogged in the inner cavity of the outer needle. However, they can be pushed out by inner needle or the like. So the outer needle may be used by itself as a bone puncture needle.

When the outer needle 11 of Figure 6a and Figure 6b are used as a bone puncture needle without use of the inner needle, the engaging groove of the rear end is also not necessary as shown in Figure 7e. Since, the tip 26a of the blade edge portion in the outer needle 11 is brought near to the long axis C compared with an usual bone puncture needle, the needle can be rotated about the needle tip. Hence, it can puncture stably also contributing to safety. Moreover, when a mark to follow the direction of the needle tip is provided, it can further contribute to safety.

Figure 8 shows a temporal fixing method of the corotation mechanism 20 provided in the above rear end. As described above, the length of the flat portion 22 of the inner needle 12 is longer than the length of the engaging groove 17 of the outer needle 11. Hence, when the inner needle 12 is inserted into the outer needle 11 and the flat portion 22 is engaged with the engaging groove 17, the back part 22a of the flat portion 22 protrudes from the rear end. And, a shrink tube 33 made of resin is fitted in the rear end of the outer needle 11 and the shrink tube 33 is shrunk by applying the heat. Thereby, the back part 22a of the flat portion 22 protruded from the rear end of the outer needle is held by the shrink tube 33, and the inner needle 12 will be unextractable from the outer needle 11. By employing such a temporal fixing means, the integration of the inner needle 12 and the outer needle 11 is made secure in either the case of when the double needle is operated by hand or when it is operated by an electrical power. In the case of extracting the inner needle 12, the inner needle may be extracted with the shrink tube or inner needle may be extracted after cutting the shrink tube. Meanwhile, in place of the shrink tube 33, an elastic tube can be used exerting a degree of effect in this case also.

The temporal fixing structure 34 shown in Figure 9a and Figure 9b comprises a male screw 35 formed in the rear end of the outer needle 11 and a nut or cap 37 equipped with a female screw 36 screwing together with the male screw 35. In the center of the inside of the cap 37, the rear end of the inner needle 12 is fixed. The cap 37 can be formed from a metal which is weldable or adherent, or from synthetic resin. The rear end of the inner needle 12 may be fixed to the cap 37, but it may also be coupled so as to be rotatable and not to come off.

Such temporal fixing structure 34 can not be employed in the case of the double needle 25 in which the vicinity of the tip 27 is made to be a whirl stop such as Figure 3a, Figure 3b, and it can be employed in the case that the inner needle 12 rotates in the outer needle 11 as the front edge side of the double needle 10 of Figure 1. But, in the case where the rear end of the inner needle 12 is coupled to the cap 37 rotatably and being fixed not to come off, the temporal fixing structure 34 may be employed even in the double needle having the whirl stop mechanism in the front edge side. The inner needle 12 is fixed to the cap 37 by welding or adhering. Then, the tip of the inner needle 12 and the outer needle 11 are made to form one plane in the position where the screw stops. In stead of fixing the cap 37 to the inner needle 12, the inner needle 12 and the cap 37 can be integrally molded.

In the temporal fixing structure 34 of Figure 9a and Figure 9b, the inner needle 12 and the outer needle 11 are integrated and exerts the effect of corotation when the cap 37 is screwed into the male screw 35 strongly to fix temporally. The puncture operation is carried out by attaching a handle or coupled to a rotation drive shaft in this state. When it is coupled to the rotation drive shaft, the rotative direction should be made to be same as the direction of tightening the screw. In the case when the inner needle 12 is extracted from the outer needle 11, the screw of the cap 37 is loosened and extracted like shown in Figure 9b.

In the temporal fixing structure 40 shown in Figure 10, a screw member 41 is fixed by crowning to the rear end of the outer needle 11, and the female screw 36 of the cap 37 is screwed together with the male screw 42 provided in the screw member 41. The screw member 41 may be made of metal and of synthetic resin also. In addition, the screw of the screw member 41 may be female screw and the cap 37 side can be made to be male screw also.

In the temporal fixing structure 42 shown in Figure 11, the protrusion 43a of an engaging member 43 fixed to the rear end of the outer needle 11 and a recessed portion 44 of the cap 37 are fitted with a snap. The reference numeral 45 is an annular protrusion which elastically engages with an annular groove 46 formed in the inside face of the cap 37. The protrusion and the recess of the engaging member 43 and the cap 37 may be reversed. The annular protrusion 45 can be composed of, for example, a separate part such as a snap ring made of metal wires etc.

The bone puncture needle 50 having tubular shape shown in Figure 12a comprises a blade edge portion 52 cut aslant by a first plane 51 at the front edge, and a blade portion 54 cut by a second plane 53 different to the first plane 51 at the tip of the blade portion. In this bone puncture needle 50, the second plane 53 is a plane inclined oppositely to the first plane 51 about the longitudinal axis (long axis) C of the puncture needle. The angle of inclination of the first plane 51 is approximately 20-40 degrees about the long axis, but in contrast, that of the second plane 53 is approximately 5-90 degrees about the long axis.

Further in this embodiment, as shown in Figure 12b, the second plane 53 is inclined in the plane view to the long axis C about 45-90 degrees, preferably about 70-80 degrees. Thereby, a linear blade portion 54 whose edge is the cross line of the first plane 51 and the second plane 53 is formed in the tip of the blade edge portion 52. In this embodiment, no step is particularly provided between the blade edge portion 52 of the bone puncture needle 50 and a body portion 56. But, the blade edge portion 52 may be made somewhat wider than the sack body 56 or may be made into somewhat elliptic shape by press forming. The outer diameter of the body portion is same as the outer needle 11 of Figure 1 etc. for example, about 2-3mm, and the inner diameter is about 1-2mm. As the material, such metals as stainless, nitinol, titanium alloy and the like may be used.

Since in this puncture needle 50 the blade portion 54 of Figure 12b is inclined, the blade edge portion 54 can scrape a cortical bone when it is rotated toward the direction of the arrow head L. Hence, rotating the puncture needle 50 by an electric drill or hand drill etc. in the direction of the arrow head L enables to puncture and scrape a cortical bone etc efficiently. Further, since the tip is made at a blunt angle, there is less danger of damaging the inside of the cortical bone opposite side to the cortical bone punctured into the bone marrow cavity, compared with the case of outer needle 11 of Figure 1. Furthermore, since the thin portion of the tip is scraped, the probability of the falling off of the portion is reduced. The second plane and the blade portion 54 may be perpendicular to the long axis C, and in this case there is the least possibility of damaging the cortical bone from inside which is in the opposite side to the cortical bone punctured in the bone marrow cavity (see Figure 14).

The bone puncture needle 57 shown in Figure 13 is equipped with the shape that is shaved off at a slant by the third plane 58 which is symmetric about the long axis C of the second plane 53 in addition to be shaved off by the second plane 53. This bone puncture needle 57 needs to be additionally processed compared with the bone puncture needle 50 of Figure 12a, 12b. However, it exerts the effect similar to that of the bone puncture needle 50 of Figure 12a, Figure 12b. Moreover, when the bone puncture needle 57 is rotated by an electric mechanical drill or hand mechanical drill etc. the rotation in any direction can scrape cortical bones etc. efficiently.

The bone puncture needle 60 shown in Figure 14 is equipped with a shape that the tip is shaved off by the second plane 61 which is perpendicular to the long axis C. This exerts such effects as preventing the damaging of the cortical bone of opposite side in the bone marrow cavity and the falling off of the tip. Further it can be easily punctured by using mechanical drills etc. and the manufacturing of this bone puncture needle is easy.

In the bone puncture needle 62 shown in Figure 15a-c, the vicinity 63 of the tip is formed to be the shape of rough elliptical shape as shown in Figure 15c. And the blade is formed by grinding in the symmetrical plane 64 of the elliptical cylinder, in other words, the plane inclined toward somewhat clockwise against the plane including the long axis of the ellipse and the long axis C of the cylinder. Hence, the elliptical annular inclined plane 65 formed by grinding is shifted right (in the figure, upward) at its tip side, and shifted left (in the figure, downward) at its hand grip side. Further as shown in Figure 15b, the inclined plane 65 can be also seen in a side view. Thereby, cortical bones can be efficiently scraped by the inside edge 66 of the elliptical annular inclined plane. Additionally, by rotating this bone puncture needle 62 in the direction toward the arrow head L1 versus the long axis C, the edge 66 can efficiently scrape at the periphery far from the long axis C, which is at the front side and at the base end side.

The bone puncture needle 67 shown in Figure 16 is equipped with a drill engaging portion 68 of rough rectangular cross section for tucking with the chuck of the drill in the vicinity of the rear of the body portion 56 or in the midway of the body portion 56. In the case that the wall thickness of the body portion 56 is thick, such drill engaging portion 68 can be formed by cutting the surface of the body portion. In this case, the internal cavity is kept in a circular cross section. However, the outer shape is usually modified into rectangular cross section by press forming because the wall thickness of the sack body 56 is thin. In this case the internal cavity is also deformed into a rectangular shape.

In the bone puncture needle 67 equipped with such drill engaging portion 68, the drill engaging portion 68 can be firmly griped by the chuck of the drill, and can prevent the slipping compared with the case that the body portion of the circular cross section is gripped as it is. Hence, cortical bones etc. can be scraped with a strong torque. The shape of the cross section of the outer face of the drill engaging portion 68 is dependent upon the shape of the drill chuck, but it is not limited to a square shape, but triangular, hexagon etc. various rectangular, elliptical shapes etc may be used. Moreover, it can be used for drills equipped with a socket type chuck or a drill bit.

Next, referencing Figure 17, the embodiment of the bone marrow harvesting method of this invention is described. In this method, a bone marrow harvesting device comprising a Kirschner wire 70, an electrical drill 71 to grip and rotate the Kirschner wire 70, a catheter 72 serving also as a needle (needle catheter), a coupling 73 to couple the Kirschner wire 70 and the needle catheter 72, and an adapter (boast adapter) 75 to couple a syringe 74 for aspiration to the rear end of the needle catheter 72. As the Kirschner wire 70, the electric drill 71, and the syringe 74, those which are publicly known can be used. The electric drill 71 is a through-type one to grip the midway of the Kirschner wire 70 and the needle catheter 72.

For the needle catheter 72, material substantially same as the above described double needle (the reference numeral 11 etc, of Figure 1-11) and the bone puncture needle (those of Figure 12-16 etc.) may be used, and the length of which is preferable to be about 100-600mm, particularly about 200-400mm, and is preferable to be equipped with a certain degree of flexibility. Further, the rear end or the vicinity of the rear of the needle catheter 72 is made to have a shape which can couple detachably an adapter 75 for coupling the syringe 74 with airtightness. Specifically, an O-ring groove to fit an O-ring for maintaining airtightness is provided, and a male screw to fix the adapter 75 is formed in the periphery, or a composition to tighten by a soft material is provided. It is preferable to provide the drill engaging portion 68 shown in Figure 16 in the midway of the needle catheter 72. The Kirschner wire 70 is preferable to be longer than the needle catheter 72 for about 250mm (150mm + equivalent length of the knob). When a boast adapter having a structure capable of maintaining airtightness is used as the adapter 75, the processing to the needle catheter 72 is not necessary, which is more preferable.

The adapter 76 shown in Figure 18a is what is the modification of the boast adapter, and comprises a rough cylindrical body 77, a tightening cap 78 to be screwed together into the periphery of the front edge of the body 77, and a packing 79 housed in a packing housing portion 77a formed in the front end of the body 77. The inside face of the packing housing portion 77a is formed to be tapered. The packing 79 presents a rough trapezoid shape in its cross section and the periphery is tapered to fit the inside face of the packing housing portion 77. In the center of the packing 79, a through hole 79a to tighten the needle catheter 72 is formed. In the center of the tightening cap 78, a hole 78a to pass through the needle catheter 72 is formed, and in the inside bottom, an annular protrusion 78b to push in the packing 79 is formed.

In the center of the body 77, a hole 77b to fit with the needle catheter 72 is formed. The hole 77b continues to a communicating hole 77d for passing through the bone marrow aspirated through the engaging step 77c engaging with the rear end of the needle catheter 72. Further, in the rear of the body 77, a cylindrical coupling portion 77e to couple with a syringe 74A is provided. In the center of the coupling portion 77e, a fit hole 77f provided with a tapered face to fit in the front edge portion 74b of the syringe 74A is formed. The front edge of the fit hole 77f is communicated with the communicating hole 77d. Further, in this embodiment, a male screw 77g to screw together with a coupling screw 74c of the syringe 74A is provided in the periphery of the coupling portion 77e. The male screw 77g is preferable to be a multithread screw such as a two thread screw.

To hold the needle catheter 72 by the adapter 76 composed as above, first, the screw of the tightening cap 78 is loosened recovering the original shape of the packing 78 by its elasticity, and the needle catheter 72 is passed through the hole 78a of the tightening cap 78, the through hole 79a of the packing 79, and the hole 77b of the body 77. And by tightening the tightening cap 78, the annular protrusion 78b pushes the packing 79 into the depth of the tapered face. Thereby, the packing 79 is reduced in its diameter, and the needle catheter 72 in the through hole 79a can be tightened up. On this occasion, the periphery of the packing 79 and the inside face of the packing housing portion 77a also contact with the state of airtightness.

The front edge of the syringe 74A is fitted in the fit hole 77f of the coupling portion 77e of the rear of the body 77, and the coupling screw 74c is screwed together with the male screw 77g. Thereby, the mutual tapered faces fit tightly to keep the airtightness. Thus, a bone marrow can be aspirated into the syringe 74A through the needle catheter 72 by operating the syringe 74a, after coupling the needle catheter 72 to the syringe 74a through the adapter 76. The adapter 76, as shown in Figure 18b, is also usable when a normal syringe 74 not equipped with the coupling screw is coupled to the needle catheter. In this case, the front edge 74a of the syringe 74 is only fitted closely in the fit hole 77f. Since the inside of the syringe 74 becomes to be negative pressure, this fitting is enough, but it may be fixed by a rubber tube or shrink tube etc. if necessary. The male screw 77g is not particularly necessary, if the normal syringe 74 does not have the coupling screw.

The coupler 73 coupling the needle catheter 72 and the Kirschner wire 70 has a cylindrical shape. The coupler 73 comprises a first holding portion to hold the vicinity of the end portion of the needle catheter so as not to rotate and not to shift toward the axial direction in its one end, and a second holding portion to hold the Kirschner wire so as not to rotate and not to shift toward the axial direction, in its another end. As the first holding portion and the second holding portion, those similar to the drill chucks can be employed. The first holding portion may be made to be the female screw which is screwed together with the male screw formed in the vicinity of the rear end of the needle catheter 72. The coupler can be formed by the structure same as the temporal fixing structure shown in Figure 8-11. Further, the coupler 73 may be a cylindrical member composed of the member from soft to elastic or thermal contraction material, as shown in Figure 19. In this case, since the configuration of the holding member is unnecessary, it can be composed by only cutting a tube. In this case, one end of the tube is a first coupling portion and another end is a second coupling portion. The first coupling portion and the second coupling portion can be formed into different diameters.

In the bone marrow harvesting method of Figure 17, first, the Kirschner wire 70 is attached to the electric drill 71, and the Kirshcner wire 70 is fed in from an epiphysis portion 80 while being rotated, then the puncturing is carried out until the front edge reaches the inside of a bone marrow cavity 81 (the first process S1). In this case, the length F from the surface of the epiphysis to the front edge of the Kirshcner wire 70 is made to be about 150mm as a target.

Then, the needle catheter 72 is attached to the electric drill 71, and the puncturing is carried out until it reaches the bone marrow cavity 81 under the guide of the Kirschner wire 70 (second process S2). In other words, the Kirshcner wire is used as a guide wire. The rear end of the Kirshcner wire 70 is kept to be pinched by one's fingers so that the Kirschner wire does not advance freely into the depth. When the electric drill 71 is used, only the needle catheter 72 rotates and the Kirshcner wire does not rotate. The Kirschner wire 70 is made to be protruded about 10-100mm from the front edge of the needle catheter 72.

Then, the rear end of the needle catheter 72 and the Kirschner wire 70 is coupled by using coupler 73, so as not to move relatively toward the axial direction. In this state, the Kirschner wire 70 and the needle catheter 72 are advanced while being rotated by the electric drill 71 to the depth of the bone marrow cavity 81 (the third process S3). Thus, advancing the needle catheter 72 and the Kirschner wire 70 together allows to shorten the necessary length of the Kirshcner wire 70 making the handling easy. More specifically, if only the Kirschner wire 70 is advanced previously into the depth, the needle catheter 72 cannot be guide enough unless the Kirschner wire 70 is left long in the outside. So, the necessary length of Kirshcner wire 70 must be twice or more as long as that of the wire uses with the needle catheter 72, making it hard to use. Moreover, advancing both the needle catheter 72 and the Kirshcner wire 70 together prevent the intrusion of the chips into the internal cavity of the needle catheter 72.

Then, the electric drill 71, the Kirschner wire 70, and the coupler 73 is removed leaving the needle catheter 72 only, the adapter 75 is attached to the rear end of the needle catheter 72, and, the syringe 74 is attached through the adapter 75. And then, the bone marrow is aspirated from the bone marrow cavity 81 by the syringe 74 through the needle catheter 72 (the forth process S4). Thereby, the bone marrow can be efficiently harvested.

In the case when the air layer in the needle catheter 72 blocks the aspiration, a three-way cock may be used to provide a gate for a preparative aspiration syringe. And the bone marrow harvesting work can be carried out by switching to the primary syringe 74, after removing the air layer by the preparative syringe. Thereby the aspiration work can be efficiently carried out.

Depending on the bone from which the bone marrow is harvested, there is a case that the path to the bone marrow 81 or the bone marrow cavity 81 is somewhat curved. In this case, it is preferable to use material such as employing a pipe having thin wall made of metal, for example, that made of nitinol, as the catheter 72 serving also as a needle,

In the above embodiment of the bone marrow harvesting method, the bone marrow harvesting device comprising the Kirshcner wire 70, the electric drill 71, the needle catheter 72, the coupler 73, syringe 74, and the adapter 75 is used. Among them, parts other than the electric drill 71, those are the Kirshcner wire 70, the electric drill 71, the needle catheter 72, the coupler 73, syringe 74, and the adapter 75, are preferable to be sold as a bone marrow harvesting kit or a bone marrow harvesting set which is a combined state of whole. Moreover, since a general purpose items can be used for the Kirschner wire 70 and the syringe 74, the needle catheter 72 and the adapter 75 can be sold as a kit. Further, the Kirschner wire 70 which is made to be of suitable length may be included in the kit.

In place of the Kirschner wire 70, a guide line or a guide wire provided with the similar functions can be used. And, a pipe shape bone puncture needle can be used as the needle catheter 72 same as described above. Further, in place of the combination of the needle catheter 72 and the Kirschner wire 70, a double needle (see Figure 1 etc.) provided with a pipe shape outer needle and an inner needle of stick shape attached inside thereof may be employed. In this case, a catheter for aspirating the bone marrow by being passed through the through hole formed by the double needle and a syringe attached to the catheter are used. Additionally, if the outer needle and the inner needle of the double needle are made long, the aspiration syringe may be attached to the outer needle directly or through the adapter, and the bone marrow can be harvested without use of the catheter.

In the case when a catheter for bone marrow aspiration is used, a bone marrow harvesting equipment comprising a bone puncture needle to be punctured to a bone marrow cavity, a catheter to aspirate the bone marrow, and a guide wire to guide the catheter along the formed through hole to the bone marrow cavity may be sold as a one set. Further, this set can also comprise a Kirschner wire.

For the needle catheter 72, the outer needle, the inner needle, the Kirschner wire 70, and the guide wire, those capable of being rotated by an electric drill are preferable. However a handle to rotate the above manually may be provided detachably. When a guide wire is used, it is preferable to bend or curve the front edge portion 85 of the guide wire 84 protruding from the front edge opening of the catheter 83 or the catheter serving also as a needle as shown in Figure 20. This can change the direction of the guide wire 84 easily by rotating the rear end of the guide wire 84.

In the guide wire 86, 87 shown in Figure 21a and Figure 21b, a spherical portion 88 is provided in front edge of both. The spherical portion 88 of the guide wire 86 of Figure 21a presents hemisphere of the diameter being same as the outer diameter of the guide wire. Hence, it is easy to insert into the catheter. On the other hand, in the guide wire 87 of Figure 21b, the diameter of the spherical portion 88 is slightly larger than the outer diameter of the guide wire. Hence, it is hard to be held up in a bone marrow cavity and easily to be advanced to the depth of the bone marrow cavity.

Since these guide wires 86, 87 are provided with the spherical portion 88 in their front edge, they cannot dig cortical bones. Hence, it is previously passed through a through hole formed by a puncture needle etc. and are used to guide a catheter. Since the guide wire 86 has a spherical portion in its front edge, it prevents from damaging the wall even if it hits the opposite side wall, after it is inserted into a bone marrow cavity.

Further, as the catheter for aspirating a bone marrow, as shown in Figure 22, a double lumen type catheter 91 is preferable, in which comprises a first lumen 89 to feed liquid such as physiologic saline in the bon marrow cavity and a second lumen 90 to aspirate the bone marrow from the bone marrow cavity. Thereby, it is possible to aspirate the bone marrow by the syringe 74 coupled to the second lumen 90 while feeding a diluted solution such as physiologic saline etc. in the bone marrow cavity.

## Claims

1. A double needle for medical treatment, comprising;
a cylindrical outer needle having a body portion and a blade edge portion at a front edge of the body portion,
a solid inner needle detachably housed in the outer needle,
wherein a rotating diameter of the blade edge portion is larger than a rotating diameter of the body portion when the outer needle is rotated about its long axis.

2. A double needle for medical treatment according to claim 1,
wherein a tip of the outer needle is tilt toward a rotation axis or a vicinity of the rotation axis in a long axis of the outer needle.

3. A double needle for medical treatment according to claim 1,
further comprising a corotation mechanism to corotate the outer needle and the inner needle when the outer needle is fitted with the inner needle and the outer needle is rotated about the long axis.

4. A double needle for medical treatment according to any of claim 1 to 3,
wherein the outer needle and the inner needle are movable along the long axis, and an engaging means to engage the outer needle and the inner needle in the rotative direction is engaged.

5. A double needle for medical treatment according to claim 1,
further comprising a mark showing the direction of the blade edge face.

6. A bone puncture needle for medical treatment, comprising;
a body portion and a blade edge portion having a cylindrical shape in a front edge of the body portion,
wherein a rotation diameter of the blade edge portion is larger than the rotation diameter of the body portion when the outer needle is rotated about its long axis.

7. A bone puncture needle for medical treatment according to claim 6,
wherein a tip of the outer needle is tilt toward a rotation axis or a vicinity of the rotation axis in a long axis of the outer needle.

8. A bone puncture needle for medical treatment according to claim 6 or claim 7,
further comprising a mark showing the direction of the blade edge face.

9. A bone puncture needle having cylindrical shape for medical treatment, comprising;
a body portion and a blade edge portion trimmed with an inclined plane at a vicinity of a front edge of the body portion,
wherein a sharpening portion trimmed with a plane other than the inclined plane is provided at a front edge of the blade edge portion.

10. A bone puncture needle for medical treatment according to claim 9,
wherein the sharpening portion provided at the front edge of the blade edge portion is trimmed with a plane perpendicular to the longitudinal axis.

11. A bone puncture needle for medical treatment according to claim 9,
wherein the sharpening portion provided at the front edge of the blade edge portion is trimmed with a plane inversely inclined to the inclined plane.

12. A bone puncture needle for medical treatment according to claim 6 (9),
wherein a vicinity of the front edge (blade edge portion) presents a crashed cylindrical shape so as to have a cross section of rough ellipse,
wherein the inclined plane is somewhat inclined to a plane including a longitudinal axis of the cylinder and the long axis of the cross section.

13. A bone puncture needle for medical treatment according to claim 9 to 12,
further comprising an aspiration port mounting portion mounting an aspiration port at the vicinity of a rear of the body portion.

14. A bone puncture needle for medical treatment according to claim 9 to 12,
further comprising a drill-engaging portion having cross section of non-circular shape for attaching a mechanical drill at a periphery of the body portion.

15. A bone marrow harvesting device, comprising;
a bone puncture needle punctured into a bone marrow,
an adapter detachable to the bone puncture needle for attaching an aspiration syringe to the bone puncture needle.

16. An adapter to communicate a bone puncture needle with the aspiration syringe, comprising;
a mounting portion detachably mounted to the bone puncture needle puncturing a bone marrow cavity, and
a mounting portion mounted to the aspiration syringe.

17. A bone marrow harvesting device according to claim 15,
further comprising, a guide wire puncturing into a bone marrow cavity prior to the bone puncture needle,
wherein the guide wire punctures a hole thinner than the bone puncture needle, and guides the bone puncture needle to the bone marrow cavity at the time of puncturing the bone puncture needle.

18. A bone marrow harvesting device, comprising;
a double needle punctured into a bone marrow cavity having a outer needle with the shape of pipe and a inner needle with the shape of stick housed in the outer needle,
a adapter detachable to the outer needle for attaching an aspiration syringe to the outer needle.

19. A bone marrow harvesting device, comprising;
a bone puncture needle to be punctured into a bone marrow cavity,
a catheter to aspirate the bone marrow, and
a guide wire to guide the catheter to the bone marrow cavity along a formed through hole to the bone marrow cavity.

20. A bone marrow harvesting device according to claim 19,
wherein the bone puncture needle is a double needle comprising an outer needle having pipe shape and an inner needle having stick shape inserting into the outer needle.

21. A bone marrow harvesting device according to claim 15, 17, 18, or 19,
wherein the bone puncture needle is attachable to a mechanical drill.

22. A bone marrow harvesting device according to claim 21,
further comprising a handle provided attachably on the bone puncture needle for rotating the bone puncture needle.

23. A bone marrow harvesting device according to claim 19 or 20,
wherein a tip of the guide wires is bent or curved for allowing the guide wire to turn in the bone marrow cavity.

24. A bone marrow harvesting device according to claim 19 or 20,
wherein a tip of the guide wire is formed in rough spherical.

25. A bone marrow harvesting device according to claim 19 or 20,
wherein the catheter comprises a first lumen feeding a liquid into a bone marrow cavity and a second lumen aspirating a bone marrow from the bone marrow cavity.

26. A method for harvesting the bone marrow, comprising;
puncturing a Kirschner wire into a bone marrow cavity,
inserting a catheter which also serves as a needle into the bone marrow cavity with the guide of the Kirschner wire,
extracting the Kirschner wire leaving the catheter,
aspirating the bone marrow through the catheter.

27. A bone marrow harvesting device, comprising;
a Kirschner wire,
a catheter which also serves as a needle guided by the Kirschner wire,
a coupling means coupling the Kirschner wire and the catheter so as not to rotate mutually, and an aspirator connected to the catheter.
